(19) Europäisches Patentamt
European Patent Office
Office européen des brevets

(11) Veröffentlichungsnummer: **0 120 276**
**B1**

(12) # EUROPÄISCHE PATENTSCHRIFT

(45) Veröffentlichungstag der Patentschrift: **15.10.86**

(21) Anmeldenummer: **84101710.6**

(22) Anmeldetag: **20.02.84**

(51) Int. Cl.⁴: **C 07 D 249/08**, C 07 D 233/60, C 07 D 403/06, A 61 K 31/41, A 61 K 31/415 // C07D405/06, C07C49/00

(54) **Substituierte Di-bzw. Triazolylalkyl-carbinole, Verfahren zu ihrer Herstellung und ihre Verwendung als antimykotische Mittel.**

(30) Priorität: **02.03.83 DE 3307218**

(43) Veröffentlichungstag der Anmeldung: **03.10.84 Patentblatt 84/40**

(45) Bekanntmachung des Hinweises auf die Patenterteilung: **15.10.86 Patentblatt 86/42**

(84) Benannte Vertragsstaaten: **AT BE CH DE FR GB IT LI LU NL SE**

(56) Entgegenhaltungen:
**EP-A-0 044 605**
**EP-A-0 069 442**
**EP-A-0 071 568**
**FR-A-2 015 913**
**FR-A-2 434 154**

**JOURNAL OF MEDICINAL CHEMISTRY, Band 12, 1969, Seiten 784-791, American Chemical Society, Easton, Pennsylvania, US; E.F. GODEFROI u.a.: "The preparation and antimycotic properties of derivatives of 1-phenethylimidazole"**
**JOURNAL OF MEDICINAL CHEMISTRY, Band 24, Nr. 6, 1981, Seiten 727-731, American Chemical Society, Easton, Pennsylvania, US; D. NARDI u.a.: "Synthesis and anticonvulsant activity of N-(benzoylalkyl)imidazoles and N-(omega-phenyl-omega-hydroxyalkyl)imidazoles"**

(73) Patentinhaber: **BAYER AG, Konzernverwaltung RP Patentabteilung, D-5090 Leverkusen 1 Bayerwerk (DE)**

(72) Erfinder: **Elbe, Hans- Ludwig, Dr., Dasnöckel 59, D-5600 Wuppertal 11 (DE)**
Erfinder: **Regel, Erik, Dipl.- Ing., Untere Bergerheide 26, D-5600 Wuppertal 1 (DE)**
Erfinder: **Büchel, Karl Heinz, Prof. Dr., Dabringhausener Strasse 42, D-5093 Burscheid (DE)**
Erfinder: **Schaller, Klaus, Dr., Am Sonnenschein 38, D-5600 Wuppertal 1 (DE)**
Erfinder: **Plempel, Manfred, Dr., Zwengenbergstrasse 3c, D-5657 Haan (DE)**

(56) Entgegenhaltungen: (Fortsetzung)
**Die Akte enthält technische Angaben, die nach dem Eingang der Anmeldung eingereicht wurden und die nicht in dieser Patentschrift enthalten sind.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 120 276 B1

LIBER, STOCKHOLM 1986

**Beschreibung**

Die vorliegende Erfindung betrifft neue substituierte Di- bzw. Triazolylalkyl-carbinole, ein Verfahren zu ihrer Herstellung und ihre Verwendung als Antimykotika.

Es ist bereits bekannt geworden, daß bestimmte Diazolyl-Derivate antimykotische Eigenschaften aufweisen. Insbesondere sei hier auf EP—A 0 044 605 hingewiesen. Die dort beschriebenen Verbindungen haben antimykotische Wirkungen.

Es wurden neue substituierte Di- bzw. Triazolylalkyl-carbinole der allgemeinen Formel

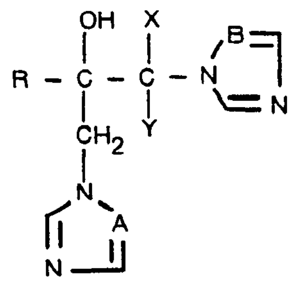

(I)

in welcher

A für ein Stickstoffatom oder die CH-Gruppe;

B für ein Stickstoffatom oder die CH-Gruppe;

X für Wasserstoff und geradkettiges oder verzweigtes Alkyl mit 1 bis 6 Kohlenstoffatomen;

Y für geradkettiges oder verzweigtes Alkyl mit 1 bis 6 Kohlenstoffatomen; sowie für den Fall, daß X für Wasserstoff steht, auch für geradkettiges oder verzweigtes Alkenyl und Alkinyl mit jeweils 3 bis 6 Kohlenstoffatomen sowie für gegebenenfalls einfach bis dreifach gleich oder verschieden im Phenylteil substituiertes Benzyl, wobei als Substituenten genannt seien:

Halogen, Alkyl mit 1 bis 4 Kohlenstoffatomen, Alkoxy und Alkylthio mit jeweils 1 bis 4 Kohlenstoffatomen, Halogenalkyl, Halogenalkoxy und Halogenalkylthio mit jeweils 1 bis 2 Kohlenstoff- und 1 bis 5 gleichen oder verschiedenen Halogenatomen, wie vorzugsweise Fluor- und Chloratomen, Nitro und Cyano;

R für gegebenenfalls einfach bis dreifach, gleich oder verschieden substituiertes Phenyl, wobei als Substituenten vorzugsweise genannt seien: Halogen, Alkyl mit 1 bis 4 Kohlenstoffatomen, Alkoxy und Alkylthio mit jeweils 1 bis 4 Kohlenstoffatomen, Halogenalkyl, Halogenalkoxy und Halogenalkylthio mit jeweils 1 bis 2 Kohlenstoff- und 1 bis 5 gleichen oder verschiedenen Halogenatomen, wie vorzugsweise Fluor- und Chloratomen, Nitro, Cyano, Hydroxy, Hydroxycarbonyl, Alkoxycarbonyl mit 1 bis 4 Kohlenstoffatomen im Alkylteil, Hydroximinoalkyl mit 1 bis 4 Kohlenstoffatomen, Alkoximinoalkyl mit 1 bis 4 Kohlenstoffatomen in jedem Alkylteil, sowie jeweils gegebenenfalls durch Halogen und/oder Alkyl mit 1 bis 2 Kohlenstoffatomen substituiertes Phenyl, Phenoxy, Benzyl und Benzyloxy; R steht ferner vorzugsweise für die Gruppierung

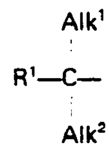

wobei

$Alk^1$ für geradkettiges oder verzweigtes Alkyl mit 1 bis 4 Kohlenstoffatomen steht;

$Alk^2$ für geradkettiges oder verzweigtes Alkyl mit 1 bis 4 Kohlenstoffatomen steht;

$Alk^1$ und $Alk^2$ gemiensam mit dem Kohlenstoffatom, an das die gebunden sind, für einen 3- bis 7-gliedrigen cycloaliphatischen Ring stehen; und

$R^1$ für geradkettiges oder verzweigtes Alkyl mit 1 bis 6 Kohlenstoffatomen, für Alkenyl mit 2 bis 4 Kohlenstoffatomen, sowie für jeweils gegebenenfalls im Phenylteil einfach bis dreifach, gleich oder verschieden substituiertes Phenyl, Phenylalkyl mit 1 bis 4 Kohlenstoffatomen im Alkylteil, Phenoxy, Phenylthio, Phenoxyalkyl mit 1 bis 4 Kohlenstoffatomen im Alkylteil, Phenylthioalkyl mit 1 bis 4 Kohlenstoffatomen im Alkylteil, Benzyloxy und Benzylthio steht, wobei als Substituenten vorzugsweise die bei R bereits genanntent Phenylsubstituenten in Frage kommen,

und deren physiologisch verträgliche Säureadditions-Salze gefunden.

Die Verbindungen der Formel (I) besitzen gegebenenfalls zwei asymmetrische Kohlenstoffatome. In diesem Falle können sie in zwei geometrischen Isomerenformen vorliegen.

Weiterhin wurde gefunden, daß man die substituierten Di- bzw. Triazolylalkyl-carbinole der Formel (I) erhält, wenn man Azolyl-oxirane der Formel

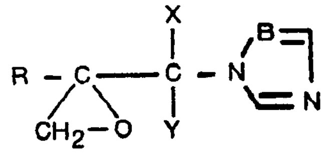

(II)

in welcher B, R, X und Y die oben angegebene Bedeutung haben, mit Azolen der Formel

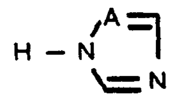

(III)

in welcher A die oben angegebene Bedeutung hat, in Gegenwart eines Verdünnungsmittels und gegebenenfalls in Gegenwart einer Base umsetzt.

An die so erhaltenen Verbindungen der Formel (I) kann gegebenenfalls anschließend eine Säure addiert werden.

Die neuen substituierten Di- bzw. Triazolylalkyl-carbinole der Formel (I) weisen starke antimykotische Eigenschaften auf.

Außerdem sind die neuen substituierten Diazolylalkylcarbinole der Formel (I) interessante Zwischenprodukte. So können z.B. die Verbindungen der allgemeinen Formel (I) an der Hydroxygruppe in üblicher Weise in die entsprechenden Ether überführt werden. Weiterhin können durch Umsetzung mit z.B. Acylhalogeniden oder Carbamoylchloriden in prinzipiell bekannter Weise Acyl- oder Carbamoyl-Derivate der Verbindungen der allgemeinen Formel (I) erhalten werden.

Außerdem können die Verbindungen der allgemeinen Formel (I), in denen $R^1$ für jeweils gegebenenfalls substituiertes Phenylthio, Phenylthioalkyl oder Benzylthio steht, in üblicher Weise zu den entsprechenden SO- bzw. $SO_2$-Derivaten oxidiert werden.

Die erfindungsgemäßen substituierten Di- bzw. Triazolylalkylcarbinole sind durch die Formel (I) allgemein definiert.

Bevorzugt sind diejenigen Verbindungen der Formel (I), in denen

A für ein Stickstoffatom oder die CH-Gruppe steht;

B für ein Stickstoffatom oder die CH-Gruppe steht;

X für Wasserstoff und geradkettiges oder verzweigtes Alkyl mit 1 bis 4 Kohlenstoffatomen steht;

Y für geradkettiges oder verzweigtes Alkyl mit 1 bis 4 Kohlenstoffatomen steht; sowie für den Fall, daß X für Wasserstoff steht, auch für Allyl, Methylallyl, Propargyl, Methylpropargyl oder für gegebenenfalls einfach bis zweifach, gleich oder verschieden im Phenylteil substituiertes Benzyl steht, wobei als Substituenten genannt seien: Fluor, Chlor, Brom, Methyl, Isopropyl, tert.-Butyl, Methoxy, Methylthio, Trifluormethyl, Trifluormethoxy, Trifluormethylthio, Nitro und Cyano;

R für gegebenenfalls einfach bis zweifach, gleich oder verschieden substituiertes Phenyl steht, wobei als Substituenten genannt seien: Fluor, Chlor, Brom, Methyl, Isopropyl, tert.-Butyl, Methoxy, Methylthio, Trifluormethyl, Trifluormethoxy, Trifluormethylthio, Nitro, Cyano, Hydroxy, Hydroxycarbonyl, Methoxycarbonyl, Ethoxycarbonyl, Hydroxyiminomethyl, 1-Hydroximinoethyl, Methoximinomethyl, 1-Methoximinoethyl, sowie jeweils gegebenenfalls durch Fluor, Chlor, Methyl substituiertes Phenyl, Phenoxy, Benzyl und Benzyloxy; ferner R für die Gruppierung

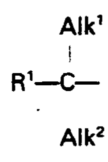

steht, wobei

$Alk^1$ für Methyl oder Ethyl steht;

$Alk^2$ für Methyl oder Ethyl steht;

$Alk^1$ und $Alk^2$ gemeinsam mit dem Kohlenstoffatom, an das sie gebunden sind, für Cyclobutyl, Cyclopentyl oder Cyclohexyl stehen; und

$R^1$ für Methyl, Ethyl, n-Propyl, i-Propyl, n-Butyl, Neopentyl, sowie für jeweils gegebenenfalls im Phenylteil einfach bis zweifach, gleich oder verschieden substituiertes Phenyl, Benzyl, Phenethyl, Phenoxy, Phenylthio, Phenoxymethyl, Phenoxyethyl, Phenylthiomethyl, Phenylthioethyl, Benzyloxy oder Benzylthio steht, wobei als Substituenten die bei R bereits genannten Phenylsubstituenten in Frage kommen.

Bevorzugte erfindungsgemäße Verbindungen sind auch Additionsprodukte aus Säuren und denjenigen substituierten Di- bzw. Triazolylalkyl-carbinolen der Formel (I), in denen die Substituenten A, B, X, Y und R die Bedeutungen haben, die bereits vorzugsweise für diese Substituenten genannt wurden.

Zu den Säuren, die addiert werden können, gehören vorzugsweise Halogenwasserstoffsäuren, wie z.B. Chlorwasserstoffsäure und die Bromwasserstoffsäure insbesondere die Chlorwasserstoffsäure, ferner Phosphorsäure, Salpetersäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sobinsäure und Milchsäure, sowie Sulfonsäuren, wie o-Toluolsulfonsäure und 1,5-Naphthalindisulfonsäure.

Verwendet man beispielsweise 2-(4-Chlorphenyl-2-[2-(1,2,4-triazol-1-yl)-2-propyl]-oxiran und 1,2,4-Triazol als Ausgangsstoffe, so kann der Ablauf des erfindungsgemaßen Verfahrens durch das folgende Formelschema wiedergegeben werden:

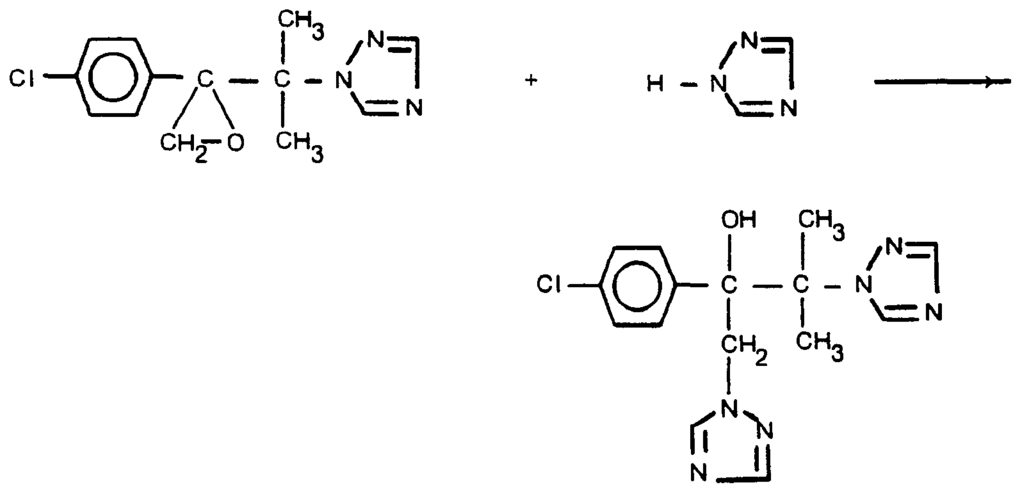

Die für die Durchführung des erfindungsgemäßen Verfahrens als Ausgangsstoffe zu verwendenden Azolyl-oxirane sind durch die Formel (II) allgemein definiert. In dieser Formel stehen B, R, X und Y vorzugsweise für die Bedeutungen, die bereits im Zusammenhang mit der Beschreibung der erfindungsgemäßen Stoffe der Formel (I) vorzugsweise für diese Substituenten genannt wurden.

Die Azolyl-oxirane der Formel (II) sind noch nicht bekannt. Sie können jedoch erhalten werden, indem man Azolyl-ketone der Formel

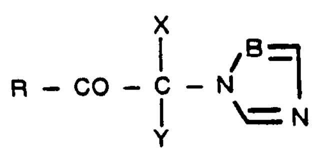

(IV)

in welcher B, R, X und Y die oben angegebene Bedeutung haben, entweder

α) mit Dimethyloxosulfonium-methylid der Formel

$$\overset{\delta +}{(CH_3)_2SO}\overset{\delta -}{CH_2} \qquad (V)$$

in an sich bekannter Weise in Gegenwart eines Verdünnungsmittels, wie beispielsweise Dimethylsulfoxid, bei Temperaturen zwischen 20°C und 80°C umsetzt (vgl. hierzu die Angaben in J. Am. Chem. Soc, *87*, 1363—1364 (1965)),

oder

β) mit Trimethylsulfonium-methylsulfat der Formel

$$[(CH_3)_3S^+]\ CH_3SO_4^- \qquad (VI)$$

in an sich bekannter Weise in Gegenwart eines inerten organischen Lösungsmittel, wie z.B. Acetonitril, und in Gegenwert einer Base, wie z.B. Natriummethylat, bei Temperaturen zwischen 0°C bis 60°C, vorzugsweise bei Raumtemperatur, umsetzt (vgl. auch die Angaben in Heterocycles *8*, 397 (1977)).

Die so erhaltenen Oxirane der Formel (II) können gegebenenfalls ohne Isolierung direkt weiter umgesetzt werden.

Die Azolyl-ketone der Formel (IV) sind teilweise bekannt (vgl. beispielsweise DE—A—2 431 407, DE—A—2 610 022, DE—A—2 628 470 und DE—A—3 048 266). Noch nicht bekannt sind Azolyl-ketone der Formel

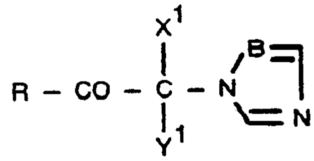

(IVa)

in welcher

$X^1$ für geradkettiges oder verzweigtes Alkyl mit 1 bis 4 Kohlenstoffatomen steht,

$Y^1$ für geradkettiges oder verzweigtes Alkyl mit 1 bis 4 Kohlenstoffatomen steht und

B für ein Stickstoffatom steht und R die oben angegebene Bedeutung hat. Eine Reihe von Vabindungen der Formel (IVa), in denen B für eine —CH-Gruppe steht, ist u.a. aus FR—A—2 434 154 oder FR—A—2 015 913 sowie J. Med Chem. *12*, (1969), s. 785 und *24*, (1981), s. 728 bekannt.

Die Azolyl-ketone der Formel (IVa) werden erhalten, indem man Halogenketone der Formel

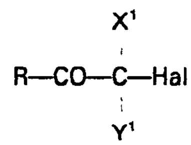

(VII)

in welcher R, $X^1$ und $Y^1$ die oben angegebene Bedeutung haben und Hal für Halogen, insbesondere Chlor oder Brom steht, mit Azolen der Formel

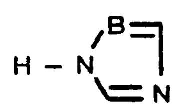

(VIII)

in welcher B die oben angegebene Bedeutung hat, in Gegenwart eines Verdünnungsmittels und in Gegenwart eines Säurebindemittels umsetzt.

Für das Verfahren zur Herstellung der Azolyl-ketone der Formel (IVa) kommen als Verdünnungsmittel inerte organische Lösungsmittel in Frage. Hierzu gehören vorzugsweise Ketone, wie Aceton und Methylethylketon; Nitrile wie Acetonitril und Propionitril; Alkohole, wie Ethanol oder Isopropanol; Ether, wie Tetrahydrofuran oder Dioxan; aromatische Kohlenwasserstoffe, wie Toluol, Benzol oder Chlorbenzol; Formamide, die Dimethylformamid und halogenierte Kohlenwasserstoffe, wie Methylenchlorid.

Das Verfahren zur Herstellung der Azolyl-ketone der Formel (IVa) wird in Gegenwart eines Säurebindemittels vorgenommen. Man kann alle üblicherweise verwendbaren anorganischen und organischen Säurebinder zugeben, wie Alkalicarbonate, beispielsweise Natriumcarbonat, Kaliumcarbonat und Natriumhydrogencarbonat, oder wei niedere tertiäre Alkylamine, Cycloalkylamine oder Aralkylamine, beispelsweise Triethylamin, N,N-Dimethylcyclohexylamin, Dicyclohexylamin, N,N-Dimethylbenzylamin, weiterhin Pyridin und Diazabicyclooctan. Vorzugsweise verwendet man einen entsprechenden überschuß an Azol.

Die Reaktionstemperaturen können beim Verfahren zur Herstellung der Azolyl-ketone der Formel (IVa) in einem größeren Bereich variiert werden. Im allgemeinen arbeitet man zwischen etwa 20 bis 150°C, vorzugsweise bei 40 bis 100°C.

Bei der Durchführung des Verfahrens zur Herstellung der Azolyl-ketone der Formel (IVa) setzt man auf 1 Mol Halogenketon der Formel (VIII) vorzugsweise 1 bis 4 Mol Azol und 1 bis 4 Mol Säurebinder ein. Die Isolierung der Azolylketone der Formel (IVa) erfolgt in üblicher Art und Weise.

Die Halogenketone der Formel (VII) sind teilweise bekannt (vgl. Synth. Commun. *12*, (1982), S. 261—266), bzw. können sie in allgemein bekannter Art und Weise erhalten werden, indem man z.B. die entsprechenden Ketone der Formel

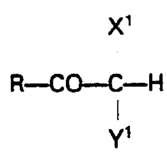

(IX)

in welcher R, $X^1$ und $Y^1$ die oben angegebene Bedeutung haben, mit Chlor oder Brom in Gegenwart eines inerten organischen Lösungsmittels, wie beispielsweise chlorierten Kohlenwasserstoffen, bei Raumtemperatur umsetzt; oder mit üblichen Chlorierungsmitteln, wie beispielsweise Sulfurylchlorid, bei Temperaturen zwischen 20 und 60°C umsetzt.

Die neuen Azolyl-ketone der Formel (IVa) stellen nicht nur interessante Zwischenprodukte dar, sondern zeigen auch selbst gute antimykotische Eigenschaften. In bestimmten Aufwandmengen können Azolylketone der Formel (IVa) auch als Pflanzenschutzmittel eingesetzt werden.

Als Verdünnungsmittel kommen für das erfindungsgemäße Verfahren unter den Reaktionsbedingungen inerte organische Lösungsmittel in Frage. Hierzu gehören vorzugsweise Alkohole, wie z.B. Ethanol, Methoxyethanol oder Propanol; Ketone, wie z.B. 2-Butanon; Nitrile, wie z.B. Acetonitril; Ester, wie z.B. Ethylacetat; Ether, wie z.B. Dioxan; aromatische Kohlenwasserstoffe, wie z.B. Benzol und Toluol; oder Amide, wie z.B. Dimethylformamid.

Als Basen kommen für die erfindungsgemäße Umsetzung alle üblicherweise verwendbaren anorganischen und organischen Basen in Betracht. Hierzu gehören vorzugsweise Alkalicarbonate, wie z.B. Natrium- und Kaliumcarbonat; Alkalihydroxide, wie z.B. Natriumhydroxid; Alkalialkoholate, wie z.B. Natrium- und Kalium-methylate und -ethylate; Alkalihydride, wie z.B. Natriumhydrid; sowie niedere tertiäre Alkylamine, Cycloalkylamine und Aralkylamine, wie insbesondere Triethylamin.

Die Reaktionstemperaturen können bei der Durchführung des erfindungsgemäßen Verfahrens in einem größeren Bereich variiert werden. Im allgemeinen arbeitet man bei Temperaturen zwischen 0 und 200°C, vorzugsweise zwischen 60 und 150°C.

Bei der Durchführung des erfindungsgemäßen Verfahrens setzt man vorzugsweise auf 1 Mol Oxiran der Formel (II) 1 bis 2 Mol Azol der Formel (III) und gegebenenfalls 1 bis 2 Mol Base ein; die Isolierung der Endprodukte erfolgt in allgemein üblicher Weise.

Die Reaktionstemperaturen können bei der Durchführung der erfindungsgemäßen Oxidation in einem größeren Bereich variiert werden. Im allgemeinen arbeitet man zwischen etwa −50 bis 100°C, vorzugsweise zeischen −30 und 80°C.

Die erfindungsgemäßen Verbindungen der Formel (I) können auch erhalten werden, indem man Di- bzw. Triazolyl-ketone der Formel

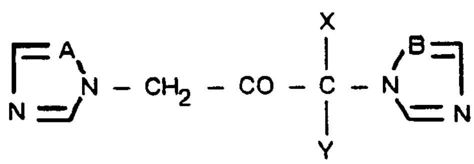

(X)

in welcher A, B, X und Y die oben angegebene Bedeutung haben, mit einem Grignard-Reagenz der Formel

R—Mg—Hal′ (XI)

in welcher R die oben angegebene Bedeutung hat und Hal′ für Halogen steht, in üblicher Weise unter den Bedingungen einer Grignard-Reaktion umsetzt; oder indem man Dihalogenalkanole der Formel

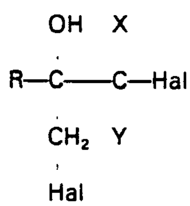

(XII)

in welcher R, Hal, X und Y die oben angegebene Bedeutung haben, in üblicher Weise mit Azolen der Formel (III) umsetzt.

Die Säureadditionssalze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem organischen Lösungsmittel gereinigt werden.

Die erfindungsgemäß verwendbaren Verbindungen der Formel (I) und ihre Säureadditions-Salze weisen antimikrobielle, insbesondere starke antimykotische Wirkungen auf. Sie besitzen ein sehr breites antimykotisches Wirkungsspektrum, insbesondere gegen Dermatophyten und Sproßpilze sowie biphasische Pilze, z.B. gegen Candida-Arten, wie Candida albicans, Epidermophyton-Arten, wie Epidermophyton floccosum, Aspergillus-Arten, wie Aspergillus niger and Aspergillus fumigatus, Trichophyton-Arten, wie Trichophyton mentagrophytes, Microsporon-Arten, wie Microsporon felineum sowie Torulopsis-Arten, wie Torulopsis glabrata. Die Aufzählung dieser Mikroorganismen stellt keinesfalls eine Beschränkung der bekämpfbaren Keime dar, sondern hat nur erläuternden Charakter.

Als Indikationsgebiete in der Humanmedizin können beispielsweise genannt werden:

Dermatomykosen und Systemmykosen durch Trichophyton mentagraphytes und andere Trichophytonarten, Microsporonarten, Epidermophyton floccosum, Sproßpilze und biphasische Pilze sowie Schimmelpilze hervorgerufen.

Als Indikationsgebiete in der Tiermedizin können beispielsweise aufgeführt werden:

Alle Dermatomykosen und Systemmykosen, insbesondere solche, die durch die obengenannten Erreger hervorgerufen werden.

Zur vorliegenden Erfindung gehören pharmazeutische Zubereitungen, die neben nicht toxischen, inerten pharmazeutisch geeigneten Trägerstoffen einen oder mehrere erfindungsgemäße Wirkstoffe enthalten oder die aus einem oder mehreren erfindungsgemäßen Wirkstoff bestehen sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entspricht. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht toxischen, inerten pharmazeutisch geeigneten Trägerstoffen sind feste, halbfeste oder flüssige Verdünnungsmittel Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotionen, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumcarbonat, (e) Lösungsverzögerer, z.B. Paraffin und (f) Resorptionsbeschleuniger, z.B. quaternäre Ammoniumverbindungen, (g) Netzmittel, z.B. Cetylalkohol, Glycerinmonostearat, (h) Adsorptionsmittel, z.B. Kaolin und Bentonit und (i) Gleitmittel, z.B. Talkum, Calcium- und Magnesiumstearat und feste Polyethylenglykole oder Gemische der unter (a) bis (i) aufgeführten Stoffe.

Den Tabletten, Dragees, Kapseln, Pillen und Granulate können mit den üblichen gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil der Intestinaltraktes, gegebenenfalls verzögert abgeben, wobei als Einbettungsmassen, z.B. Polymersubstanzen und Wachse verwendent werden können.

Der oder die Wirkstoffe können gegebenenfalls mit einem oder mehreren der oben angegebenen Trägerstoffen auch in mikroverkapselter Form vorliegen.

Suppositorein können neben dem oder den Wirkstoffen die üblichen wasserlöslichen oder wasserunlöslichen Trägerstoffe enthalten, z.B. Polyethylenglykole, Fette, z.B. Kakaoffet und höhere Ester (z.B. $C_{14}$-Alkohol mit $C_{16}$-Fettsäure) oder Gemische dieser Stoffe.

Salben, Pasten, Cremes oder Gele können neben dem oder den Wirkstoffen die üblichen Trägerstoffe enthalten, z.B. tierische und pflanzliche Fette, Wachse, Paraffine, Stärke, Tragant, Cellulosederivate, Polyethylenglykole, Silicone, Bentonite, Kieselsäure, Talkum oder Zinkoxid oder Gemische dieser Stoffe.

Puder und Sprays können neben dem oder den Wirkstoffen die üblichen Trägerstoffe enthalten, z.B. Milchzucker, Talkum Kieselsäure, Aluminiumhydroxid, Calciumsilikat und Polyamidpulver oder Gemische dieser Stoffe, Sprays können zusätzlich die üblichen Treibmittel z.B. Chlorfluorkohlenwasserstoffe enthalten.

Lösungen und Emulsionen können nebem dem oder den Wirkstoffen die üblichen Trägerstoffe wie Lösungsmittel, Lösungsvermittler und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutplasmaisotonischer Form vorliegen.

Suspensionen können neben dem oder den Wirkstoffen die üblichen Trägerstoffe wie flüssige Verdünnungsmittel, z.B. Wasser, Ethylalkohol, Propylenglykol, Suspendiermittel, z.B. ethoxylierte Isostearylalkohole, Polyoxyethylensorbit- und Sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbessernde Zusätze, z.B. Pfefferminzöl und Eukalyptusöl und Süßmittel, z.B. Saccharin enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-% der Gesamtmischung vorhanden sein.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise

nach bekannten Methoden, z.B. durch Mischen des oder der Wirkstoffe mit dem oder den Trägerstoffen.

Zur vorliegenden Erfindung gehören auch die Verwendung der erfindungsgemäßigen Wirkstoffe sowie von pharmazeutischen Zubereitungen, die einen oder mehrere erfindungsgemäße Wirkstoffe enthalten, in der Human- und Veterinärmedizin zur Verhütung, Besserung und/oder Heilung der oben angeführten Erkrankungen.

Die Wirkstoffe oder die pharmazeutischen Zubereitungen können lokal, oral, parenteral, intraperitoneal und/oder rektal, vorzugsweise parenteral, insbesondere intravenös appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 10 bis etwa 300, vorzugsweise 50 bis 200 mg/kg Körpergewicht je 24 Stunden, gegebenenfalls in Form mehrerer Einzelgaben zur Erzielung der gewünschten Ergebnisse zu verabreichen.

Es kann jedoch erforderlich sein, von denen genannten Dosierungen abzuweichen und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichens sein, mit weniger als der obengenannten Menge Wirkstoff auszukommen, während in anderen Fällen die oben angeführte Wirkstoffmenge überschritten werden muß. Die Festlegung der jeweils erforderlichen opitmalen Dosierung und Applikationsart der Wirkstoffe kann durch jeden Fachmann aufgrund seines Fachwissens leicht erfolgen

## Herstellungsbeispiele

### Beispiel 1

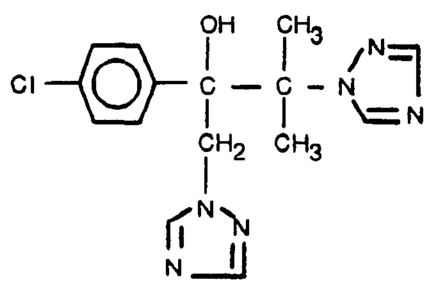

Zu einer Lösung von 0,11 g (0,047 Mol) Natrium in 30 ml n-Propanol werden bei Raumtemperatur unter Rühren 3,6 g (0,052 Mol) 1,2,4-Triazol gegeben. Man erhitzt auf Rückflußtemperatur und versetzt mit einer Lösung bon 2-(4-Chlorphenyl)-2-[2-(1,2,4-triazol-1-yl)-prop-2-yl]-oxiran in 20 ml n-Propanol. Das Reaktionsgemisch wird 15 Stunden unter Rückfluß erhitzt, danach abgekühlt und auf Wasser gegeben. Man extrahiert mit Methylenchlorid, trocknet die organische Phase über Natriumsulfat und engt ein. Der Rückstand wird säulenchromatographisch (Kieselgel; Chloroform:Methanol = 9:1) gereinigt. Man erhält 3,7 g (24,2% der Theorie) 2-(4-Chlorphenyl)-1,3-di-(1,2,4-triazol-1-yl)-3-methyl-2-butanol vom Schmelzpunkt 114°C.

### Herstellung des Ausgangsproduktes

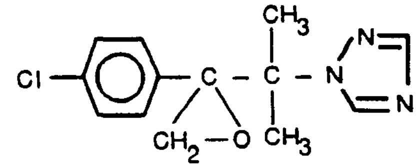

9,6 g (0,044 Mol) Trimethylsulfoniumodid werden unter Stickstoffatmosphäre in 9,6 g (0,118 Mol) Dimethylsulfat gelöst. Bei Raumtemperatur gibt man 5,2 g (0,044 Mol) Kalium-tert.-butylat zu und läßt 6 Stunden bei Raumtemperatur nachrühren. Anschließend wird bei Raumtemperatur eine Lösung von 9,9 g (0,0397 Mol) 4-Chlorphenyl-[2-(1,2,4-triazol-1-yl)-prop-2-yl]-keton in 16 ml Tetrahydrofuran zugetropft. Man läßt das Reaktionsgemisch 15 Stunden bei Raumtemperatur und 4 Studen unter Rückfluß nachrühren, kühlt ab und filtriert. Das Filtrat wird im Vakuum eingeengt, der Rückstand auf Wasser gegeben und mit Methylenchlorid extrahiert. Die organische Phase wird über Natriumsulfat getrocknet und im Vakuum eingeengt. Man erhält 10,4 g (100% der Theorie) 2-(4-Chlorphenyl-2-[2-(1,2,4-triazol-1-yl)-prop-2-yl]-oxiran vom Brechungsindex $n_D^{20} = 1{,}5388$.

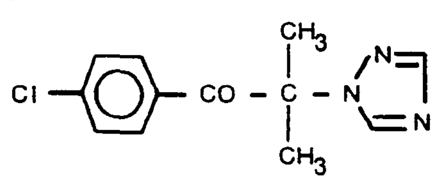

(IVa–1)

80 g (0,31 Mol) (2-Brom-prop-2-yl)-4-chlorophenyl-keton, 26,9 g (0,39 Mol) 1,2,4-Triazol und 53,8 g (0,39 Mol) Kaliumcarbonat werden in 350 ml Aceton 6 Stunden unter Rückfluß erhitzt. Man läßt abkühlen, filtriert und engt das Filtrat im Vakuum ein. Der Rückstand wird in Methylenchlorid aufgenommen, mit Wasser gewaschen, über Natriumsulfat getrocknet und im Vakuum eingeengt. Der Rückstand wird aus Diisopropylether umkristallisiert.

Man erhält 19,9 g (25,7% der Theorie) 4-Chlrophenyl-[2-(1,2,4-triazol-1-yl)-prop-2-yl]-keton vom Schmelzpunkt 111°C.

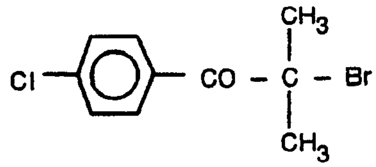

65,5 g (0,36 Mol) 4-Chlorphenyl-isopropyl-keton werden in 200 ml Chloroform gelöst und mit 1 ml Bromwasserstoff/Eisessig versetzt. Man läßt dann bei 30°C 57,5 g (0,36 Mol) Brom langsam zutropfen und 30 Minuten bei Raumtemperatur nachrühren. Man engt im Vakuum ein und erhält 86,6 g (92% der Theorie) rohes (2-Brom-prop-2-yl)-4-chlorophenyl-keton als Öl, das direkt weiter umgesetzt wird.

Beispiel 2

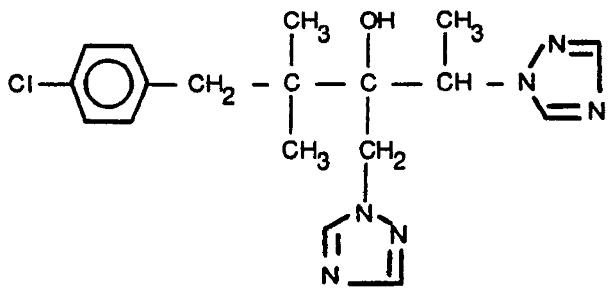

Zu einer Lösung von 0,1 g (0,043 Mol) Natrium in 30 ml n-Propanol werden bei Raumtemperatur unter Rühren 3,6 g (0,052 Mol) 1,2,4-Triazol gegeben. Man versetzt danach mit einer Lösung von 13,2 g (0,043 Mol) 2-(4-Chlorphenyl-tert.-butyl)-2-[1-(1,2,4-triazol-1-yl)-ethyl]-oxiran in 20 ml n-Propanol. Das Reaktionsgemisch wird 20 Stunden unter Rückfluß erhitzt, dann abgekühlt und auf 300 ml Wasser gegeben. Man extrahiert mit Methylenchlorid, wäscht die Methylenchloridphase mit Wasser, trocknet über Natriumsulfat und engt im Vakuum ein. Der Rückstand wird säulenchromatographisch (Kieselgel; Essigester:Ether = 3:1) gereinight. Man erhält 4 g (24,8% der Theorie) 1-(4-Chlorophenyl)-2,2-dimethyl-3-(1,2,4-triazol-1-yl)-methyl)-4-(1,2,4-triazol-1-yl)-3-pentanol vom Schmelzpunkt 59°C.

Herstellung des Ausgangsproduktes

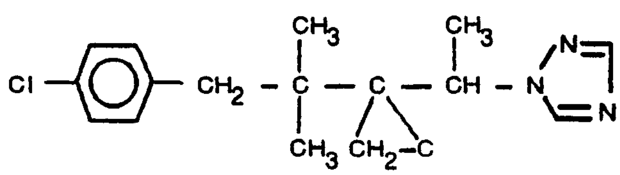

Eine Lösung von 9,8 g (0,77 Mol) Dimethylsulfat und 5,3 g (0,085 Mol) Dimethylsulfid in 50 ml Acetonitril läßt man 5 Tage bei Raumtemperatur rühren. Anschließend läßt man bei Raumtemperatur eine Lösung von 13 g (0,044 Mol) (4-Chlorphenyl-tert.-butyl)-[1-(1,2,4-triazol-1-yl)-ethyl]-keton in 20 ml Acetonitril zutropfen. Bei gleicher Temperatur trägt man 4,8 g (0,088 Mol) Natriummethylat ein, läßt 20 Stunden nachrühren und engt anschließend im Vakuum ein. Der Rückstand wird mit einem Gemisch aus 35 ml Essigester und 25 ml Wasser über Nacht verrührt. Die organische Phase wird abgetrennt, über Natriumsulfat getrocknet und im Vakuum eingeengt. Man erhält 13,2 g (98,2% der Theorie) 2-(4-Chlorphenyl-tert.-butyl)-2-[1-(1,2,4-triazol-1-yl)-ethyl]-oxiran vom Brechungsindex $n_D^{20} = 1,5431$.

In entsprechender Weise werden die folgenden Verbindungen der allgemeinen Formel

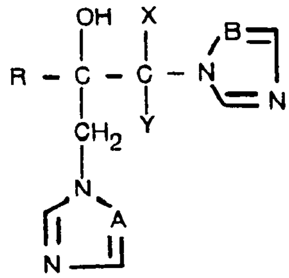

(I)

erhalten:

| Bsp. Nr. | R | X | Y | A | B | Schmelzpunkt (°C) bzw. $n_D^{20}$ |
|---|---|---|---|---|---|---|
| 3 | 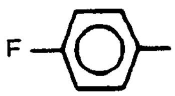 | $CH_3$ | $CH_3$ | N | N | 56 |
| 4 | 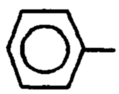 | $CH_3$ | $CH_3$ | N | N | 156 |
| 5 | 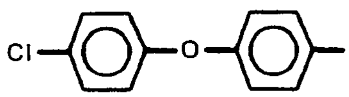 | $CH_3$ | $CH_3$ | N | N | 202 |
| 6 | 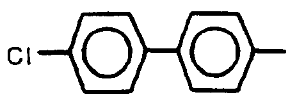 | $CH_3$ | $CH_3$ | N | N | 185–90 |
| 7 | 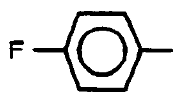 | $CH_3$ | $CH_3$ | CH | N | 196 |
| 8 | 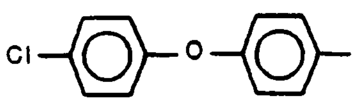 | $CH_3$ | $CH_3$ | CH | N | 90–100 |
| 9 | 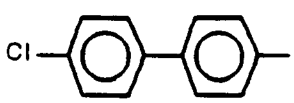 | $CH_3$ | $CH_3$ | CH | N | 100–10 |
| 10 | 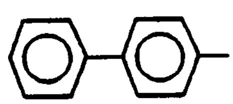 | H | $CH_3$ | N | N | glasartig |
| 11 | 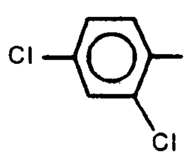 | H | $CH_3$ | N | N | 143–145 |
| 12 | $(CH_3)_3C-$ | H | $CH_3$ | N | N | 110 |

| Bsp. Nr. | R | X | Y | A | B | Schmelzpunkt (°C) bzw. $n_D^{20}$ |
|---|---|---|---|---|---|---|
| 13 | 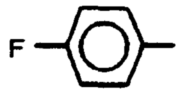 | H | $CH_3$ | N | N | 96–99 |
| 14 | 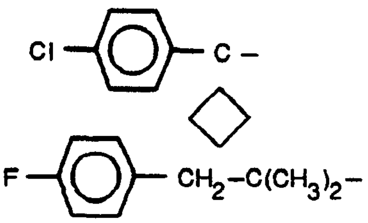 | H | $CH_3$ | N | N | 47 |
| 15 | | H | $CH_3$ | N | N | 36 |
| 16 | 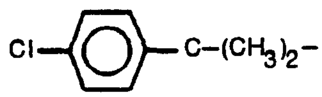 | H | $CH_3$ | N | N | 52 (Form A) |
| 17 | 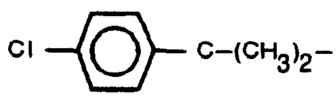 | H | $CH_3$ | N | N | 60 (Form B) |
| 18 | 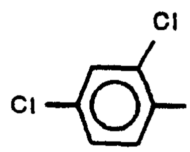 | H | 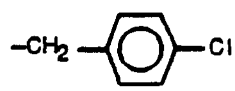 | N | N | 126 |
| 19 | ,, | H | $-CH_2-CH{=}CH_2$ | N | N | 144 |
| 20 | ,, | H | $-C_4H_5-n$ | N | N | 154 |
| 21 | ,, | H | $-C_2H_5$ | N | N | 110 |

Formen A und B: Die beiden möglichen geometrischen Isomeren Entsprechend Beispiel 1 und entsprechend den angegebenen Verfahrensbedingungen werden die folgenden Zwischenprodukte der allgemeinen Formel

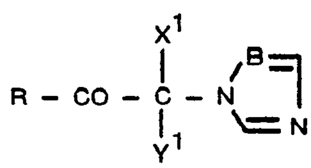

(IVa)

erhalten.

| Bsp. Nr. | R | $X^1$ | $Y^1$ | B | Schmelzpunkt (°C) bzw. $n_D^{20}$ |
|---|---|---|---|---|---|
| (IVa–2) | 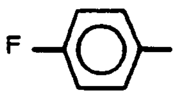 | $CH_3$ | $CH_3$ | N | 104 |
| (IVa–3) | 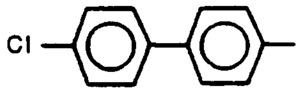 | $CH_3$ | $CH_3$ | N | 145–50 |
| (IVa–4) | 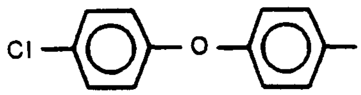 | $CH_3$ | $CH_3$ | N | 112 |
| (IVa–5) | 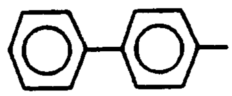 | $CH_3$ | $CH_3$ | N | 140–45 |
| (IVa–6) | 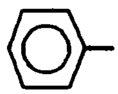 | $CH_3$ | $CH_3$ | N | 132 |
| (IVa–7) | 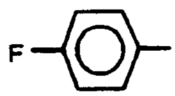 | $CH_3$ | $CH_3$ | CH | 1,5273 |
| (IVa–8) | 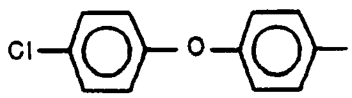 | $CH_3$ | $CH_3$ | CH | 1,5751 |
| (IVa–9) | 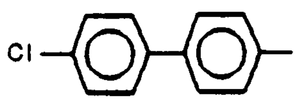 | $CH_3$ | $CH_3$ | CH | 140 |
| (IVa–10) | 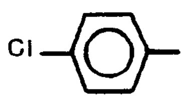 | $CH_3$ | $CH_3$ | CH | 1,5505 |
| (IVa–11) | 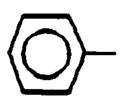 | $CH_3$ | $CH_3$ | CH | 1,5445 |
| (IVa–12) | 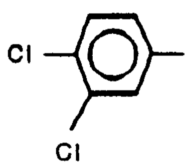 | $CH_3$ | $CH_3$ | N | |
| (IVa–13) | 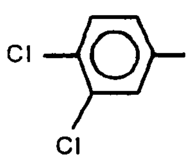 | $CH_3$ | $CH_3$ | CH | |
| (IVa–14) | 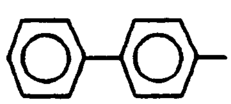 | $CH_3$ | $CH_3$ | CH | |

Beispiel A

Antimykotische in vitro-Wirksamkeit

*Versuchsbeschreibung:*

Die in-vitro-Prüfungen wurden im Reihenverdünnungstest mit Keiminokula von durchschnittlich $5 \times 10^4$ Keimen/ml Substrat durchgeführt. Als Nährmedium dienten

a) für Dermatophyten und Schimmelpilze: Sabourand's milieu d'épreuve

b) für Hefen: Fleischextrakt-Traubenzucker-Bouillon.

Die Bebrütungstemperatur betrung 20°C, die Bebrütungsdauer lag bei 24 bis 96 Stunden bei Hefen und 96 Stunden bei Dermatophyten und Schimmelpilzen.

In diesem Test zeigen insbesondere die Verbindungen der Herstellungsbeispiele 3, 5, 6, 8, 9 und 10 ein gutes antimykotisches Wirkungsspektrum.

TABELLE A

Antimykotische in-vitro-Wirksamkeit

| Wirkstoff | MHK-Werte in γ/ml Nährmedium bei Tricho-phyton mentagr. | Candida albicans | Torulop-sis glabrata | Aspergillus fumigatus |
|---|---|---|---|---|
| Verbindungen gemäß Her-stellungsbei-spiel | | | | |
| 3 | 16 | 16 | 64 | >64 |
| 5 | ≤1 | 2 | 32 | >64 |
| 6 | ≤1 | 64 | — | 16 |
| 8 | 8 | 4 | 32 | 64 |
| 9 | 4 | 8 | 8 | 32 |
| 10 | 2 | 2 | 64 | 64 |

Beispiel B

Antimykotische in-vitro-Wirksamkeit (oral) bei Mäusecandidose

*Versuchsbeschreibung:*

Mäuse vom Typ SPF—$CF_1$ wurden intravenös mit 1—2 × $10^6$ logarthmisch wachsenden Candida-Zellen, die in physiologischer Kochsalzlösung suspendiert werden, infiziert. Eine Stunde vor und sieben Stunden nach der Infektion werden die Tiere mit jeweils 10—50 mg/kg Körpergewicht der Präparate oral behandelt.

*Ergebnis:*

Unbehandelte Tiere starben 3 bis 6 Tage post infektionem. Die Überlebensrate am 6. tag post infektionem betrung bei unbehandelten Kontrolltieren etwa 5%.

In diesem Test zeigten z.B., die Verbindungen der Herstellungsbeispiele 1, 3, 4 und 5 eine gute antimykotische Wirkung.

Zeichenerklärung

| | | |
|---|---|---|
| +++++ | = sehr gute Wirkung | = 90% Überlebende am 6. Tag p.i. |
| ++++ | = gute Wirkung | = 80% Überlebende am 6. Tag p.i. |
| +++ | = Wirkung | = 60% Überlebende am 6. Tag p.i. |
| ++ | = schwache Wirkung | = 40% Überlebende am 6. Tag p.i. |
| + | = Spur Wirkung | = |
| k.W. | = keine Wirkung | |

TABELLE B
Antimykotische in-vivo-Wirksamkeit (oral) bei Mäusecandidose

| Verbindungen gemäß Herst. Beispiel | |
|---|---|
| 1 | +++++ |
| 3 | +++++ |
| 4 | +++++ |
| 5 | ++++ |

Beispiel/Formulierungen

1. Lösung

| | |
|---|---|
| Wirkstoff gemäß Formel (I): | 10 g |
| Alkohol, rein (96 %ig): | 300 g |
| Isopropylmyristat: | 526 g |
| | 836 g |

2. Creme

| | |
|---|---|
| Wirkstoff gemäß Formel (I): | 10 g |
| Arlacel 60: | 20 g |
| (Sorbitan-monostearat) Tween 60: | 15 g |
| (Polyoxyethylen (20)-sorbitan-mono-stearat) Walrat, künstlich: | 30 g |
| (Mischung von Estern von gesättigten Fettsäuren $C_{14}$—$C_{18}$ und Fettalkoholen $C_{14}$—$C_{18}$) Lanette O: | 100 g |
| (Gemisch aus Cetyl-Alkohol und Stearyl-Alkohol) Entanol G: | 135 g |
| (2-Octyl-dodecanol) Benzylalkohol: | 10 g |
| Wasser, entmineralisiert: | 680 g |
| | 1000 g |

**Patentansprüche**

1. Substituierte Di-bzw. Triazolylalkyl-carbinole der allgemeinen Formel

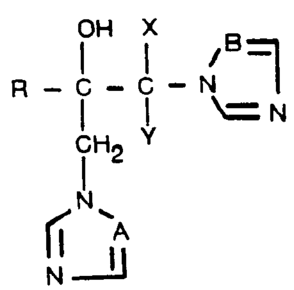

(I)

in welcher

A für ein Stickstoffatom oder die CH-Gruppe;

B für ein Stickstoffatom oder die CH-Gruppe;

X für Wasserstoff und geradkettiges oder verzweigtes Alkyl mit 1 bis 6 Kohlenstoffatomen;

Y für geradkettiges oder verzweigtes Alkyl mit 1 bis 6 Kohlenstoffatomen; sowie für den Fall, daß X für Wasserstoff steht, auch für geradkettiges oder verzweigtes Alkenyl und Alkinyl mit jeweils 3 bis 6 Kohlenstoffatomen sowie für gegebenenfalls einfach bis dreifach gleich oder verschieden im Phenylteil substituiertes Benzyl, wobei als Substituenten genannt seien: Halogen, Alkyl mit 1 bis 4 Kohlenstoffatomen, Alkoxy und Alkylthio mit jeweils 1 bis 4 Kohlenstoffatomen, Halogenalkyl, Halogenalkoxy und Halogenalkylthio mit jeweils 1 bis 2 Kohlenstoff- und 1 bis 5 gleichen oder verschiedenen Halogenatomen, wie vorzugsweise Fluor- und Chloratomen, Nitro und Cyano;

R für gegebenenfalls einfach bis driefach, gleich oder verschieden substituiertes Phenyl, wobei als Substituenten genannt seien: Halogen, Alkyl mit 1 bis 4 Kohlenstoffatomen, Alkoxy und Alkylthio mit

jeweils 1 bis 4 Kohlenstoffatomen, Halogenalkyl, Halogenalkoxy und Halogenalkylthio mit jeweils 1 bis 2 Kohlenstoff- und 1 bis 5 gleichen oder verschiedenen Halogenatomen, in form von vorzugsweise Fluor- und Chloratomen, Nitro, Cyano, Hydroxy, Hydroxycarbonyl, Alkoxycarbonyl mit 1 bis 4 Kohlenstoffatomen im Alkylteil, Hydroximinoalkyl mit 1 bis 4 Kohlenstoffatomen, Alkoximinoalkyl mit 1 bis 4 Kohlenstoffatomen, in jedem Alkylteil, sowie jeweils gegebenenfalls durch Halogen und/oder Alkyl mit 1 bis 2 Kohlenstoffatomen substituiertes Phenyl, Phenoxy, Benzyl und Benzyloxy; R steht ferner für die Gruppierung

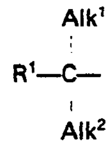

wobei

$Alk^1$ für geradkettiges oder verzweigtes Alkyl mit 1 bis 4 Kohlenstoffatomen steht;

$Alk^2$ für geradkettiges oder verzweigtes Alkyl mit 1 bis 4 Kohlenstoffatomen steht;

$Alk^1$ und $Alk^2$ gemeinsam mit dem Kohlenstoffatom, an das sie gebunden sind, für einen 3- bis 7-gliedrigen cycloalkiphatischen Ring stehen; und

$R^1$ für geradkettiges oder verzweigtes Alkyl mit 1 bis 6 Kohlenstoffatomen, für Alkenyl mit 2 bis 4 Kohlenstoffatomen, sowie für jeweils gegebenenfalls im Phenylteil einfach bis dreifach, gleich oder verschieden substituiertes Phenyl, Phenylalkyl mit 1 bis 4 Kohlenstoffatomen im Alkylteil, Phenoxy, Phenylthio, Phenoxyalkyl mit 1 bis 4 Kohlenstoffatomen im Alkylteil, Phenylthioalkyl mit 1 bis 4 Kohlenstoffatomen im Alkylteil, Benzyloxy und Benzylthio steht, wobei als Substituenten die bei R bereits genannten Phenylsubstituenten in Frage kommen.

2. Verbindungen der Formel (I) in Anspruch 1, in der

A für ein Stickstoffatom oder die CH-Gruppe steht;

B für ein Stickstoffatom oder die CH-Gruppe steht;

X für Wasserstoff und geradkettiges oder verzweigtes Alkyl mit 1 bis 4 Kohlenstoffatomen steht;

Y für geradkettiges oder verzweigtes Alkyl mit 1 bis 4 Kohlenstoffatomen steht, sowie für den Fall, daß X für Wasserstoff steht, auch für Allyl, Methylallyl, Propargyl, Methylpropargyl oder für gegebenenfalls einfach bis zweifach, gleich oder verschieden in Phenylteil substituiertes Benzyl steht, wobei also Substituenten genannt seien: Fluor, Chlor, Brom, Methyl, Isopropyl, tert.- Butyl, Methoxy, Methylthio, Trifluormethyl, Trifluoromethoxy, Trifluormethylthio, Nitro und Cyano;

R für gegebenenfalls einfach bis zweifach, gleich oder verschieden substituiertes Phenyl steht, wobei als Substituenten genannt seien: Fluor, Chlor, Brom, Methyl, Isopropyl, tert.-Butyl, Methoxy, Methylthio, Trifluormethyl, Trifluormethoxy, Trifluormethylthio, Nitro, Cyano, Hydroxy, Hydroxycarbonyl, Methoxycarbonyl, Ethoxycarbonyl, Hydroxyiminomethyl, 1-Hydroxyiminoethyl, Methoximinomethyl, 1-Methoximinoethyl, sowie jeweils gegebenenfalls durch Fluor, Chlor, Methyl substituiertes Phenyl, Phenoxy, Benzyl und Benzyloxy; ferner R für die Gruppierung

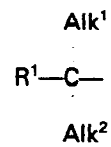

steht, wobei

$Alk^1$ für Methyl oder Ethyl steht;

$Alk^2$ für Methyl oder Ethyl steht;

$Alk^1$ und $Alk^2$ gemeinsan mit dem Kohlenstoffatom, an das sie gebunden sind, für Cyclobutyl, Cyclopentyl oder Cyclohexyl stehen; und

$R^1$ für Methyl, Ethyl, n-Propyl, i-Propyl, n-Butyl, Neopentyl, sowie für jeweils gegebenenfalls im Phenylteil einfach bis zweifach, gleich oder verschieden substituiertes Phenyl, Benzyl, Phenethyl, Phenoxy, Phenylthio, Phenoxymethyl, Phenoxyethyl, Phenylthiomethyl, Phenylthioethyl, Benzyloxy oder Benzylthio steht, wobei als Substituenten die bei R bereits genannten Phenylsubstituenten in Frage kommen.

3. 2-(4-Chlorophenyl)-1,3-di-(1,2,4-triazol-1-yl)-3-methyl-2-butanol.

4. 2-(4-Fluorphenyl)-1,3-di-(1,2,4-triazol-1-yl)-3-methyl-2-butanol.

5. 2-Phenyl-1,3-di-(1,2,4-triazol-1-yl)-3-methyl-2-butanol.

6. Azolylketone der allgemeinen Formel

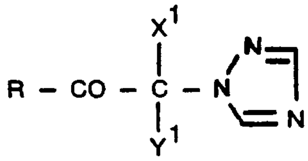

(IVa)

in welcher

$X^1$ für geradkettiges oder verzweigtes Alkyl mit 1 bis 4 Kohlenstoffatomen steht,

$Y^1$ für geradkettiges oder verzweigtes Alkyl mit 1 bis 4 Kohlenstoffatomen steht und
R die oben angegebene Bedeutung hat.

7. Substituierte Di-bzw. Triazolylalkyl-carbinole der allgemeinen Formel (I) in Anspruch 1 zur Anwendung bei der therapeutischen Behandlung des menschlichen oder tierischen Körpers.

8. Verfahren zur Herstellung von substituierten Di-bzw. Triazolylalkyl-carbinolen der allgemeinen Formel

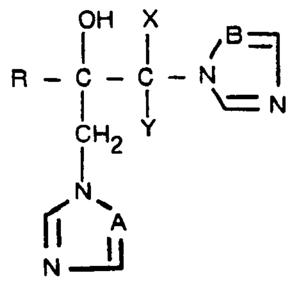

(I)

in welcher

A für ein Stickstoffatom oder die CH-Gruppe;

B für ein Stickstoffatom oder die CH-Gruppe;

X für Wasserstoff und geradkettiges oder verzweigtes Alkyl mit 1 bis 6 Kohlenstoffatomen;

Y für geradkettiges oder verzweigtes Alkyl mit 1 bis 6 Kohlenstoffatomen; sowie für den Fall, daß X für Wasserstoff steht, auch für geradkettiges oder verzweigtes Alkenyl und Alkinyl mit jeweils 3 bis 6 Kohlenstoffatomen sowie für gegebenenfalls einfach bis dreifach gleich oder verschieden im Phenylteil substituiertes Benzyl, wobei als Substituenten genannt seien: Halogen, Alkyl mit 1 bis 4 Kohlenstoffatomen, Alkoxy und Alkylthio mit jeweils 1 bis 4 Kohlenstoffatomen, Halogenalkyl, Halogenalkoxy und Halogenalkylthio mit jeweils 1 bis 2 Kohlenstoff- und 1 bis 5 gleichen oder verschiedenen Halogenatomen, wie vorzugsweise Fluor- und Chloratomen, Nitro und Cyano;

R für gegebenenfalls einfach bis dreifach, gleich oder verschieden substituiertes Phenyl, wobei als Substituenten vorzugsweise genannt seien: Halogen, Alkyl mit 1 bis 4 Kohlenstoffatomen, Alkoxy und Alkylthio mit jeweils 1 bif 4 Kohlenstoffatomen, Halogenalkyl, Halogenalkoxy und Halogenalkylthio mit jeweils 1 bis 2 Kohlenstoff- und 1 bis 5 gleichen oder verschiedenen Halogenatomen, wie vorzugsweise Fluor- und Chloratomen, Nitro, Cyano, Hydroxy, Hydroxycarbonyl, Alkoxycarbonyl mit 1 bis 4 Kohlenstoffatomen im Alkylteil, Hydroximinoalkyl mit 1 bis 4 Kohlenstoffatomen, Alkoxyiminoalkyl mit 1 bis 4 Kohlenstoffatomen, in jedem Alkylteil, sowie jeweils gegebenenfalls durch Halogen und/oder Alkyl mit 1 bis 2 Kohlenstoffatomen substituiertes Phenyl, Phenoxy, Benzyl und Benzyloxy; R steht ferner vorzugsweise für die Gruppierung

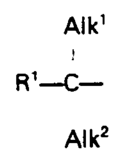

wobei

$Alk^1$ für geradkettiges oder verzweigtes Alkyl mit 1 bis 4 Kohlenstoffatomen steht;

$Alk^2$ für geradkettiges oder verzweigtes Alkyl mit 1 bis 4 Kohlenstoffatomen steht;

$Alk^1$ und $Alk^2$ gemeinsam mit dem Kohlenstoffatom, an das sie gebunden sind, für einen 3- bis 7-gliedrigen cycloaliphatischen Ring stehen; und

$R^1$ für geradkettiges oder verzweigtes Alkyl mit 1 bis 6 Kohlenstoffatomen, für Alkenyl mit 2 bis 4 Kohlenstoffatomen, sowie für jeweils gegebenenfalls im Phenylteil einfach bis dreifach, gleich oder verschieden substituiertes Phenyl, Phenylalkyl mit 1 bis 4 Kohlenstoffatomen im Alkylteil, Phenoxy, Phenylthio, Phenoxyalkyl mit 1 bis 4 Kohlenstoffatomen im Alkylteil, Phenylthioalkyl mit 1 bis 4 Kohlenstoffatomen im t 1 bis 4 Kohlenstoffatomen im Alkylteil, benzyloxy und Benzylthio steht, wobei als Substituenten vorzugsweise die bei R bereits genannten Phenylsubstituenten in Frage kommen, dadurch gekennzeichnet, daß man Azolyl-oxirane der Formel

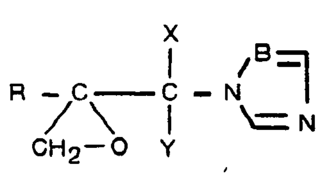

(II)

in welcher

B, R, X, und Y die oben angegebene Bedeutung haben, mit Azolen der Formel

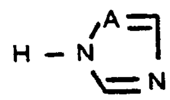

(III)

in welcher

A die oben angegebene Bedeutung hat, in Gegenwart eines Verdünnungsmittels und gegebenenfalls in Gegenwart einer Base umsetzt.

9. Arzneimittel, gekennzeichnet durch einen Gehalt an mindestens einer Verbindung gemäß Anspruch 1.

**Revendications**

1. Di- et triazolylalkylcarbinols substitués de formule générale

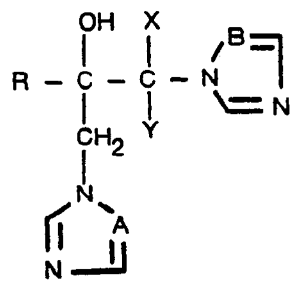

(I)

dans laquelle

A désigne un atome d'azote ou le groupe CH;

B est un atome d'azote ou le groupe CH;

X représente l'hydrogène ou un groupe alkyle à chaîne droite ou ramifié ayant 1 à 6 atomes de carbone;

Y désigne un groupe alkyle à chaîne droite ou ramifié ayant 1 à 6 atomes de carbone; de même que, au cas où X représente l'hydrogène, un groupe alcényle et un groupe alcynyle à chaîne droite ou ramifiés ayant chacun 3 à 6 atomes de carbone ainsi que, le cas échéant, un groupe benzyle portant éventuellement un à trois substituants indentiques ou différents dans la partie phényl, et on mentionne alors comme substituants: un halogène, un groupe alkyle ayant 1 à 4 atomes de carbone, des groupes alkoxy et alkylthio ayant chacun 1 à 4 atomes de carbone, des groupes halogénalkyle, halogénalkoxy et halogénalkylthio ayant chacun 1 ou 2 atomes de carbone et 1 à 5 atomes d'halogènes identiques ou différents, tels que, de préférence, des atomes de fluor et de chlore, un groupe nitro et un groupe cyano;

R désigne un groupe phényl portant éventuellement un à trois substituants identiques on différents, et on mentione alors de préférence comme substituants: un halogène, une groupe alkyle ayant 1 à 4 atomes de carbone, des groupes alkoxy et alkythio ayant chacun 1 à 4 atomes de carbone, des groupes halogénalkyle, halogènalkoxy et halogénalkylthio ayant chacun 1 ou 2 atomes de carbone et 1 à 5 atomes d'halogènes identiques ou différents, tels que, de préférence, des atomes de fluor et de chlore, un groupe nitro, cyano, hydroxy, hydroxycarbonyle, un groupe alkoxycarbonyle ayant 1 à 4 atomes de carbone dans la partie alkyle, un groupe hydroxyiminoalkyle ayant 1 à 4 atomes de carbone, alkoxyiminoalkyle ayant 1 à 4 atomes de carbone dans chaque partie alkyle, ainsi que des groupes phényle, phénoxy, benzyle or benzyloxy éventuellement substituées chacun par un halogène et/ou un radical alkyle ayant 1 ou 2 atomes de carbone; R représente en outre de préférence le groupement

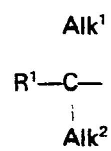

dans lequel

$Alk^1$ désigne un groupe alkyle à chaîne droite ou ramifié ayant 1 à 4 atomes de carbone;

$Alk^2$ représente un groupe alkyle à chaîne droite ou ramifié ayant 1 à 4 atomes de carbone;

$Alk^1$ und $Alk^2$ forment un noyau cycloaliphatique de 3 à 7 chaînons en commun avec l'atome de carbone auquel ils sont liés; et

$R^1$ est un groupe alkyle à chaîne droite ou ramifié ayant 1 à 6 atomes de carbone, un groupe alcényle ayant 2 à 4 atomes de carbone, ainsi que des groupes phényle, phénylalkyle ayant 1 à 4 atomes de carbone dans la partie alkyle, phénoxy, phénylthio, phényloxyalkyle ayant 1 à 4 atomes de carbone dans la partie alkyle, phénylthioalkyle ayant 1 à 4 atomes de carbone dans la partie alkyle, benzyloxy et benzylthio portant chacun, le cas échéant, dans la partie phényle un à trois substituants identiques ou différents, et on considère alors comme substituants de préférence les substituants du groupe phényle qui ont été déjà mentionnès dans le cas de R.

2. Composés de formule (I) suivant la revendication 1, dans laquelle

A désigne un atome d'azote ou le groupe CH;

B désigne un atome d'azote ou le groupe CH;

X représente l'hydrogène et un groupe alkyle à chaîne droite ou ramifié ayant 1 à 4 atomes de carbone;

Y désigne un groupe alkyle à chaîne droite ou ramifié ayant 1 à 4 atomes de carbone, ainsi que, au cas où X représente l'hydrogène, également une groupe allyle, méthylallyle, propargyle, méthylpropargyle ou un groupe benzyle portant éventuellement un ou deux substituants identiques ou différents dans la partie phényle, et on mentionne alors comme substituants: le fluor, le chlore, le brome, un radical méthyle, isopropyle, tertio-butyle, méthoxy, méthylthio, trifluorométhyle, trifluorométhoxy, trifluorométhylthio, nitro et cyano;

R désigne un groupe phényle portant éventuellement un ou deux substituants identiques ou différents, et on mentionne alors comme substituants: le fluor, le chlore, le brome, un radical méthyle, isopropyle, tertiobutyle, méthoxy, méthylthio, trifluorométhyle, trifluorométhoxy, trifluorométhylthio, nitro, cyano, hydroxy, hydroxycarbonyle, méthoxycarbonyl, éthoxycarbonyle, hydroximinométhyle, 1-hydroximinoéthyle, méthoximinométhyle, 1-méthoximinoéthyle, ainsi que des groupes phényle, phénoxy, benzyle et benzyloxy éventuellement substitués chacun par un fluor, du chlore ou un radical méthyle; en outre, R représente le groupement

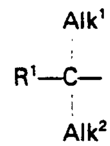

dans lequel

$Alk^1$ est une groupe méthyle ou éthyle;

$Alk^2$ est une groupe méthyle ou éthyle;

$Alk^1$ et $Alk^2$ forment conjointement avec l'atome de carbone auquel ils sont liés une groupe cyclobutyle, cyclophentyle ou cyclohexyle; et

$R^1$ est un groupe méthyle, éthyle, n-propyle, isopropyle, n-butyle, néopentyle, ainsi qu'un groupe phényle, benzyle, phénéthyle, phénoxy, phénylthio, phénoxyméthyle, phénoxyéthyle, phénylthiométhyle, phénylthioéthyle, benzyloxy ou benzylthio portant chacun, le cas échéant, dans la partie phényle un ou deux substituants identiques ou différents, et on considère alors comme substituants les substituants du groupe phényle déjà mentionnés à propos de R.

3. Le 2-(4-chlorophényl)-1,3-di-(1,2,4-triazole-1-yl)-3-méthyl-2-butanol.

4. Le 2-(4-fluorophényl)-1,3-di-(1,2,4-triazole-1-yl)-3-méthyl-2-butanol.

5. Le 2-phényl-1,3-di-(1,2,4-triazole-1-yl)-3-méthyl-2-butanol.

6. Azolylcétones de formule générale

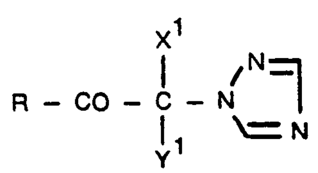

(IVa)

dans laquelle

$X^1$ est un groupe alkyle à chaîne droite un ramifié ayant 1 à 4 atomes de carbone.

$Y^1$ est un groupe alkyle à chaîne droite ou ramifié ayant 1 à 4 atomes de carbone et

R a la définition indiquée ci-dessus.

7. Di- et triazolylalkylcarbinols substitués de formule générale (I) suivant la revendication 1, destinés à être utilisés dans le traitement thérapeutique du corps humain ou animal.

8. Procédé de production de di- ou triazolyl-alkylcarbinols substitués de formule générale

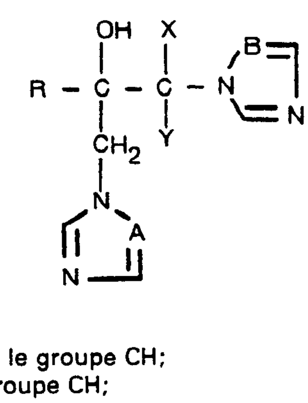

(I)

dans laquelle

A désigne un atome d'azote ou le groupe CH;

B est un atome d'azote ou le groupe CH;

X représente l'hydrogène ou un groupe alkyle à chaîne droite ou ramifié ayant 1 à 6 atomes de carbone;

Y désigne un groupe alkyle à chaîne droite ou ramifié ayant 1 à 6 atomes de carbone; de même que, au cas où X représente l'hydrogène, un groupe alcényle et un groupe alcynyle à chaîne droite ou ramifiés ayant chacun 3 à 6 atomes de carbone ainsi que, le cas échéant, un groupe benzyle portant éventuellement un à trois substituants identiques ou différents dans la partie phényle, et on mentionne alors comme substituants: un halogène, un groupe alkyle ayant 1 à 4 atomes de carbone, des groupes alkoxy et alkylthio ayant chacun 1 à 4 atomes de carbone, des groupes halogènalkyle, halogénalkoxy et halogénalkylthio ayant chacun 1 ou 2 atomes de carbone et 1 à 5 atomes d'halogènes identiques ou différents, tels que, de préférence, des atomes de fluor et de chlore, un groupe nitro et un groupe cyano;

R désigne un groupe phényle portant éventuellement un à trois substituants identiques ou différents, et on mentione alors de préférence comme substituants: un halogéne, un groupe alkyle ayant 1 à 4 atomes de carbone, des groupes alkoxy et alkylthio ayant chacun 1 à 4 atomes de carbone, des groupes halogénalkyle, halogénalkoxy et halogénalkylthio ayant chacun 1 ou 2 atomes de carbone et 1 à 5 atomes d'halogènes identiques ou différents, tels que, de préférence, des atomes de fluor et de chlore, un groupe nitro, cyano, hydroxy, hydroxycarbonyle, un groupe alkoxycarbonyle ayant 1 à 4 atomes de carbone dans la partie alkyle, un groupe hydroxyiminoalkyle ayant 1 à 4 atomes de carbon, alkoxyiminoalkyle ayant 1 à 4 atomes de carbone dans chaque partie alkyle, ainsi que des groupes phényle, phénoxy, benzyle et benzyloxy éventuellement substitués chacun par un halogène et/ou un radical alkyle ayant 1 ou 2 atomes de carbone; R représente en outre de préférence le groupement

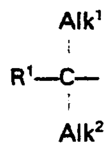

dans lequel

$Alk^1$ désigne un groupe alkyle à chaîne droite ou ramifié ayant 1 à 4 atomes de carbone;

$Alk^2$ represente un groupe alkyle à chaîne droite ou ramifié ayant 1 à 4 atomes de carbone;

$Alk^1$ et $Alk^2$ forment un noyau cycloaliphatique de 3 à 7 chaînons en commun avec l'atome de carbone auquel ils sont liès; et

$R^1$ est un groupe alkyle à chaîne droite ou ramifié ayant 1 à 6 atomes de carbone, un groupe alcényle ayant 2 à 4 atomes de carbone, ainsi que des groupes phényle, phénylalkyle ayant 1 à 4 atomes de carbone dans la partie alkyle, phénoxy, phénylthio, phénoxyalkyle ayant 1 à 4 atomes de carbone dans la partie alkyle, phénylthioalkyle ayant 1 à 4 atomes de carbone dans la partie alkyle, benzyloxy et benzylthio portant chacun, le cas échéant, dans la partie phényle un à trois substituants identiques ou différents, et on considére alors comme substituants de préférence les substituants du groupe phényle qui ont été déjà mentionnès dans le cas de R, caractérisé en ce qu'on fait réagir des azolyloxiranes de formule

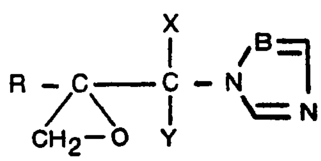

(II)

dans laquelle

B, R, X et Y ont la définition indiquée ci-dessus, avec des azoles de formule

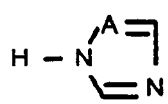

(III)

dans laquelle

A a la définition indiquée ci-dessus, en présence d'un diluant et, le cas écheant, en présence d'une base.

9. Médicaments, caractérisés par une teneur en au moins un composé suivant la revendication 1.

**Claims**

1. Substituted di- or triazolylalkyl-carbinols of the general formula

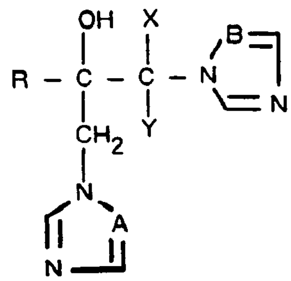

(I)

in which

A represents a nitrogen atom or the CH group;

B represents a nitrogen atom or the CH group;

X represents hydrogen and straight-chain or branched alkyl with 1 to 6 carbon atoms;

Y represents straight-chain or branched alkyl with 1 to 6 carbon atoms; and, if X represents hydrogen, also straight-chain or branched alkenyl and alkinyl with in each case 3 to 6 carbon atoms and benzyl which is optionally mono- to trisubstituted in the phenyl part by identical or different substituents, substituents which may mentioned being: halogen, alkyl with 1 to 4 carbon atoms, alkoxy and alkylthio with in each case 1 to 4 carbon atoms, halogenoalkyl, halogenoalkoxy and halogenoalkylthio with in each case 1 to 2 carbon and 1 to 5 identical or different halogen atoms, such as preferably fluorine and chlorine atoms, nitro and cyano;

R represents phenyl which is optionally mono- to tri-substituted by identical or different substituents, preferred substituents which may be mentioned being: halogen, alkyl with 1 to 4 carbon atoms, alkoxy and alkylthio with in each case 1 to 4 carbon atoms, halogenoalkyl, halogenoalkoxy and halogenoalkylthio with in each case 1 to 2 carbon and 1 to 5 identical or different halogen atoms, such as preferably fluorine and chlorine atoms, nitro, cyano, hydroxyl, hydroxycarbonyl, alkoxcarbonyl with 1 to 4 carbon atoms in the alkyl part, hydroximinoalkyl with 1 to 4 carbon atoms, alkoximinoalkyl with 1 to 4 carbon atoms in each alkyl part, and phenyl, phenoxy, benzyl and benzyloxy, each of which is optionally substituted by halogen and/or alkyl with 1 to 2 carbon atoms; R furthermore preferably represents the grouping

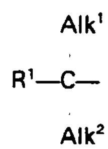

wherein

$Alk^1$ represents straight-chain or branched alkyl with 1 to 4 carbon atoms;

$Alk^2$ represents straight-chain or branched alkyl with 1 to 4 carbon atoms;

$Alk^1$ and $Alk^2$, together with the carbon atom to which they are bonded, represent a 3- to 7-centered cycloaliphatic ring; and

$R^1$ represents, straight-chain or branched alkyl with 1 to 6 carbon atoms, alkenyl with 2 to 4 carbon atoms and phenyl, phenylalkyl with 1 to 4 carbon atoms in the alkyl part, phenoxy, phenylthio, phenoxyalkyl with 1 to 4 carbon atoms in the alkyl part, phenylthioalkyl with 1 to 4 carbon atoms in the alkyl part, benzyloxy and benzylthio, each of which is optionally mono- to trisubstituted in the phenyl part by identical or different substituents, preferred possible substituents being the phenyl substituents already mentioned for R.

Compounds of the formula (I) in Claim 1, in which

A represents a nitrogen atom or the CH group;

B represents a nitrogen atom or the CH group;

X represents hydrogen and straight-chain or branched alkyl with 1 to 4 carbon atoms;

Y represents straight-chain or branched alkyl with 1 to 4 carbon atoms and, if X represents hydrogen, also represents allyl, methyallyl, propargyl, methypropargyl or benzyl which is optionally mono- to disubstituted in the phenyl part by identical or different substituents, substituents which may be mentioned being: fluorine, chlorine, bromine, methyl, isopropyl, tert.-butyl, methoxy, methylthio, trifluoromethyl, trifluoromethoxy, trifluoromethylthio, nitro and cyano;

R represents phenyl which is optionally mono- to di- substituted by identical or different substituents, substituents which may be mentioned being: fluorine, chlorine, bromine, methyl, isopropyl, tert-butyl, methoxy, methylthio, trifluoromethyl, trifluoromethoxy, trifluoromethylthio, nitro, cyano, hydroxyl, hydroxycarbonyl, methoxycarbonyl, ethoxycarbonyl, hydroximinomethyl, 1-hydroximinoethyl, methoximinomethyl, 1-methoximinoethyl, and phenyl, phenoxy, benzyl and benzyloxy, each of which is

optionally substituted by fluorine, chlorine or methyl; furthermore R represents the grouping

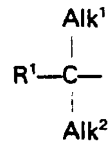

wherein

Alk[1] represents methyl or ethyl;

Alk[2] represents methyl or ethyl;

Alk[1] and Alk[2], together with the carbon atom to which they are bonded, represent cyclobutyl, cyclopentyl or cyclohexyl; and

$R^1$ represents methyl, ethyl, n-propyl, i-propyl, n-butyl, neopentyl and phenyl, benzyl, phenethyl, phenoxy, phenylthio, phenoxymethyl, phenoxyethyl, phenylthiomethyl, phenylthioethyl, benzyloxy or benzylthio, each of which is optionally mono- to disubstituted in the phenyl part by identical or different substituents, possible substituents being the phenyl substituents already mentioned for R.

3. 2-(4-Chlorophenyl)-1,3-di-(1,2,4-triazol-1-yl)-3-methyl-2-butanol.

4. 2-(4-Fluorophenyl)-1,3-di-(1,2,4-triazol-1-yl)-3-methyl-2-butanol.

5. 2-Phenyl-1,3-di-(1,2,4-triazol-1-yl)-3-methyl-2-butanol.

6. Azolyl ketones of the general formula

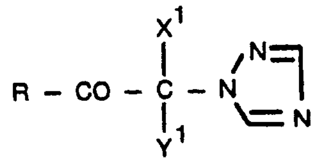

(IVa)

in which

$X^1$ represents straight-chain or branched alkyl with 1 to 4 carbon atoms,

$Y^1$ represents straight-chain or branched alkyl with 1 to 4 carbon atoms and

R has the abovementioned meaning.

7. Substituted di- or triazolylalkyl-carbinols of the general formula (I) in Claim 1 for use in the therapeutic treatment of the human or animal body.

8. Process for the preparation of substituted di- or triazolylalkyl-carbinols of the general formula

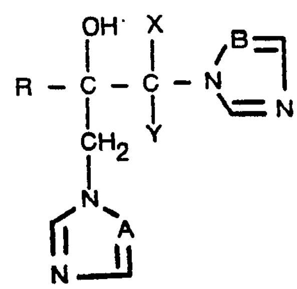

(I)

in which

A represents a nitrogen atom or the CH group;

B represents a nitrogen atom or the CH group;

X represents hydrogen and straight-chain or branched alkyl with 1 to 6 carbon atoms;

Y represents straight-chain or branched alkyl with 1 to 6 carbon atoms; and, if X represents hydrogen, also straight-chain or branched alkenyl and alkinyl with in each case 3 to 6 carbon atoms and benzyl which is optionally mono- to trisubstituted in the phenyl part by identical or different substituents, substituents which may be mentioned being: halogen, alkyl with 1 to 4 carbon atoms, alkoxy and alkylthio with in each case 1 to 4 carbon atoms, halogenoalkyl, halogenoalkoxy and halogenoalkylthio with in each case 1 to 2 carbon and 1 to 5 identical or different halogen atoms, such as preferably fluorine and chlorine atoms, nitro and cyano;

R represents phenyl which is optionally mono- to trisubstituted by identical or different substituents, preferred substituents which may be mentioned being: halogen, alkyl with 1 to 4 carbon atoms, alkoxy and alkylthio with in each case 1 to 4 carbon atoms, halogenoalkyl, halogenoalkoxy and halogenoalkylthio with in each case 1 to 2 carbon and 1 to 5 identical or different halogen atoms, such as preferably fluorine and chlorine atoms, nitro, cyano, hydroxyl, hydroxycarbonyl, alkoxcarbonyl with 1 to 4 carbon atoms in the alkyl part, hydroximinoalkyl with 1 to 4 carbon atoms, alkoximinoalkyl with 1 to 4 carbon atoms in each alkyl part and phenyl, phenoxy, benzyl and benzyloxy, each of which is optionally substituted by halogen and/or alkyl with 1 to 2 carbon atoms;

R furthermore preferably represents the grouping

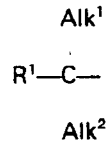

wherein

Alk[1] represents straight-chain or branched alkyl with 1 to 4 carbon atoms;

Alk[2] represents straight-chain or branched alkyl with 1 to 4 carbon atoms;

Alk[1] and Alk[2], together with the carbon atom to which they are bonded, represent a 3- to 7-membered cycloaliphatic ring; and

$R^1$ represents straight-chain or branched alkyl with 1 to 6 carbon atoms, alkenyl with 2 to 4 carbon atoms, and phenyl, phenyalkyl with 1 to 4 carbon atoms in the alkyl part, phenoxy, phenylthio, phenoxyalkyl with 1 to 4 carbon atoms in the alkyl part, phenylthioalkyl with 1 to 4 carbon atoms in the alkyl part, benzyloxy and benzylthio, each of which is optionally mono- to trisubstituted in the phenyl part by identical or different substituents, preferred possible substituents being the phenyl substituents already mentioned for R, characterised in that azolyl-oxiranes of the formula

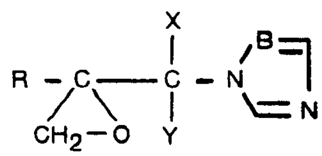

(II)

in which

B, R, X and Y have the abovementioned meaning, are reacted with azoles of the formula

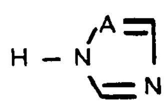

(III)

in which

A has the abovementioned meaning, in the presence of a diluent and if appropriate in the presence of a base.

9. Medicaments, characterised in that they contain at least one compound according to Claim 1.